# EUROPEAN PATENT APPLICATION

(11) **EP 3 842 541 A1**
(43) Date of publication of application: **30.06.2021**
(21) Application number: 19916540.8
(22) Date of filing: 10.01.2019
(51) Int. Cl.: C12Q 1/68, A61B 5/117, G06K 19/08, A61B 10/02, B41K 1/00

(54) **FILM FOR COLLECTING DNA AND METHOD OF OBTAINING, PURIFYING AND SEQUENCING**

(30) Priority: 10.10.2018 US 201862743753 P
(71) Applicant: Blanco, Sergio, Santiago, 7560749 (CL); Ortega, Gabriel Edgardo, Cordoba, 5003 (AR)
(72) Inventor: Blanco, Sergio, Santiago, 7560749 (CL); Ortega, Gabriel Edgardo, Cordoba, 5003 (AR)
(74) Representative: Isern Patentes y Marcas S.L.
(86) International application number: PCT/CL2019/050004
(87) International publication number: WO 2020/073140

(57) **Abstract**

A sticker with adhesive sheet for obtaining human DNA for genetic analysis or diagnosis, characterized by the fact that the sticker comprises 6 sheets arranged one above the other, comprising: a first top layer of 50 microns crystal type biaxially oriented polypropylene (BOOP); a second layer with silicone treatment; a sheet of acrylate adhesive; a third layer of 60 microns white biaxially oriented polypropylene (BOOP); a fourth layer of transparent material; a fifth layer with silicone treatment, and; a sixth layer of 50 microns crystal type biaxially oriented polypropylene (BOOP). A method for obtaining human DNA for genetic analysis or diagnosis, the method comprising the step of: providing the DNA sticker according to claim 1; extracting a sample from a user by using the DNA sticker; soaking the DNA sticker with an extraction solution from a commercially available DNA kit, and; following the manufacturer's instructions for use.

## Description

### FIELD OF THE INVENTION

The present invention relates to an extraction of DNA samples, and more specifically to an adhesive sheet or sticker and associated method for extraction of DNA samples and sequencing using human epidermal remnants to perform biomedical studies including the PCR technique for the amplification of DNA sequences, with enough quantity to obtain coding and non-coding DNA using commercial kits.

### BACKGROUND OF THE INVENTION

The genetic material DNA (deoxyribonucleic acid) constitutes the genetic material which encodes the synthesis of cellular components and the metabolism of all living beings. Different devices and methods of genetic identification have been developed for identification and filiation of people and use in forensic sciences, the DNA is obtained from blood samples or swab techniques where epithelial cells of the mouth are used, with the disadvantages that each of these processes bring according to age, state of health, religion or legal regulations (USA "Guidelines for a quality assurance program for DNA analysis", CrimeLaboratoryDigest 22, 21-43, 1995). In order to overcome these disadvantages, different adhesive devices have been developed, such as adhesive tapes for forensic medicine published in various scientific publications; "Applicability of DNA Analysis on Adhesive Tape in Forensic Casework", author Zech, WD | Malik, N | Thali, M; Univ Bern Organization | University of Bern | Univ Zurich | University of Zurich | Inst Forens Med | Univ Bern University of Bern Inst Forens Med Univ Zurich University of Zurich Inst Forens Med, Source "Journal of Forensic Sciences | 57 (4): 1036-1041 Jul 2012; Title "The Use of Adhesive Tape for Recovery of DNA from Crime Scene Items"; Author Barash, M | Reshef, A | Brauner, P; Organization Israel Police Natl Headquarters | DIFS | Forens Biol Lab | Israel Police Natl Headquarters DIFS Forens Biol Lab; Source JOURNAL OF FORENSIC SCIENCES | 55 (4): 1058-1064 JUL 2010; "Direct PCR amplification of forensic touch and other challenging DNA samples": A review; Author Cavanaugh, SE | Bathrick, AS, Org. Bode Cellmark Forensics Inc, source; FORENSIC SCIENCE INTERNATIONAL-GENETICS, Publication January 2018 and Title "Rapid multiplex DNA amplification on an inexpensive microdevice for human identification via short tandem repeat analysis"; Author DuVall, JA | You Roux, D | Thompson, BL | Birch, C | Nelson, DA | Li, JY | Mills, DL | Tsuei, A | Ensenberger, MG | Sprecher, C | Storts, DR | Root, BE | Landers, JP; Univ Virginia Organization | University of Virginia | TeGrex Technol | Promega Corp | Dept Chem | Dept Mech & Aerosp Engn | Hlth Sci Ctr | Dept Pathol | Appl Res Inst | Univ Virginia University of Virginia Dept Chem Univ Virginia University of Virginia Dept Mech & Aerosp Engn Univ Virginia University of Virginia Hlth Sci Ctr Dept Pathol TeGrex Technol Promega Corp Univ Virginia University of Virginia Appl Res Inst; Source ANALYTICA CHIMICA ACTA | 980: 41-49 AUG 8 2017. "Analysis of fingerprint samples, testing various conditions, for forensic DNA identification" author Ostojic, L | Wurmbach, E; organization Off Chief Med Examiner | Dept Forens Biol | Off Chief Med Examiner Dept Forens Biol; SCIENCE & JUSTICE font | 57 (1): 35-40 JAN 2017.

The associated methods are described in several documents found, as disclosed in Document KR326937B1, of Jeong Yeon-bo | Kim Eun-young published on March 13, 2002 and entitled "A method to obtain human skin DNA samples with an adhesive sheet"; which consists of the "use of recording a visualized image
comprising dyeing the surface of the adhesive sheet with violet glass"; in the document WO2003046210A1 entitled "Method of sampling" of AGRESEARCH LIMITED | LINGARD LABEL COMPANY LIMITED Publication 05/06/2003, in
document KR2004006621A entitled "Method for isolating DNA using diatomaceous earth from human skin collected by adhesive sheet" discloses "A method to isolate DNA using diatomaceous earth from human skin collected by an adhesive sheet" of
I.D. GENE INC. Publication January 24, 2004 ; similar to the document EP1115883B1 entitled "Method for obtaining human skin DNA samples with an adhesive sheet", of I.D. Gene Inc., publication July 28, 2004 , and finally the document EP2395338A1 entitled "Device for obtaining DNA samples" of Gentras S.r.l, publication December 14, 2011.

### BRIEF DESCRIPTION OF THE DRAWINGS

For a better clarity and understanding of the object of the present invention, it has been illustrated in several figures, in which the same has been represented in one of the preferred forms of embodiments, all by way of example, wherein:

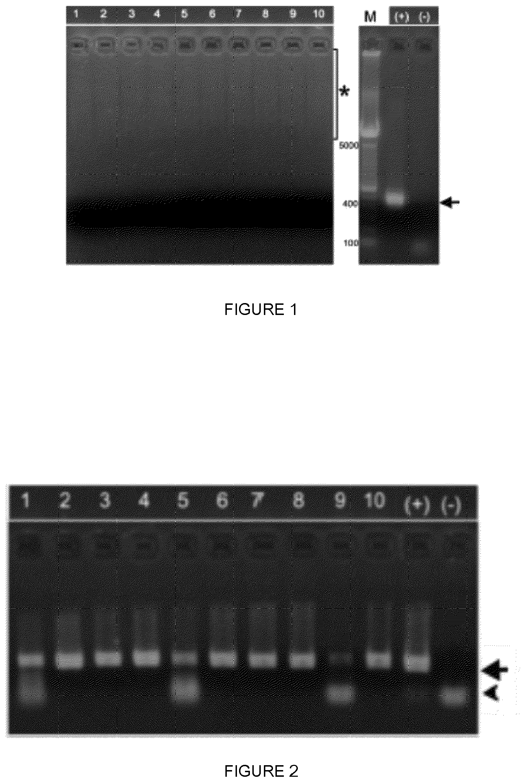

**Figure 1** is an image showing the display of DNA purification from agarose gel from multiple finger samples. In the agarose gel visualization of DNA purified from multiple finger samples. Streets 1 to 10, DNA samples from stickers with multiple fingerprints according to the detailed code in Table. M; molecular weight marker with the detail of three reference values. It is noted a continuous mark of nucleic acids above the band of 5000 bases (*). (+),positive control in PRC reaction, approximately 400 bases-arrow-. (-), negative control of PCR reaction, with a diffuse band of very low weight, corresponding to the mixture of reaction primers.
**Figure 2** is an image showing an agarose gel showing PCR reactions from multiple fingerprints. The image of agarose gel showing PCR reactions from multiple finger samples is observed. Streets 1 to 10, PCR reactions using samples of DNA extractions from stickers with multiple fingerprints (according to the code detailed in Table 1). (+), positive control of the PCR reaction, using purified genomic DNA from venous blood as target. (-), negative control of PCR using a sticker without sample as target. The upper band of 400 bases (arrow) corresponds to the expected PCR product, while the smaller diffuse band corresponds to the primers (arrowhead).

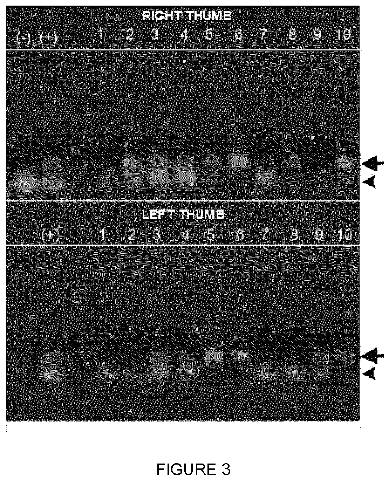

**Figure 3** is an image showing agarose gels showing PCR reactions from single thumbprint samples, where the top panel corresponds to the right thumb and the lower panel corresponds to the left thumb. Agarose gels are observed showing PCR reactions from single thumbprint samples. Top panel, right thumb and bottom panel, left thumb. For both gels, Streets; 1 to 10, PCR reactions using samples of DNA extractions from stickers with single fingerprints samples (according to the code detailed in Table 1). (+), positive control of the PCR reaction, using genomic DNA purified from venous blood as target. (-), negative PCR control using a sticker without sample as target. Both groups of reactions show positive reactions of the expected size (400 bases, arrow), the diffuse band of smaller size corresponding to the primers (arrowhead).

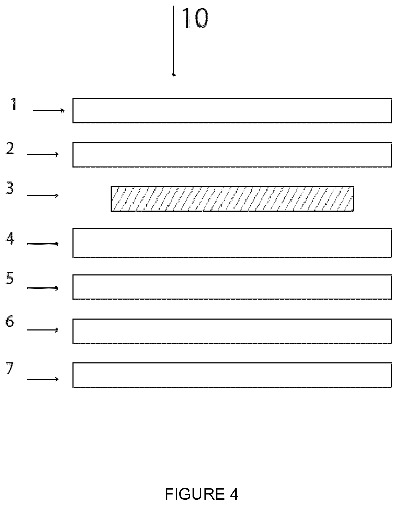

Figure 4 shows an exploded view of the sticker of the present invention showing the different layers;

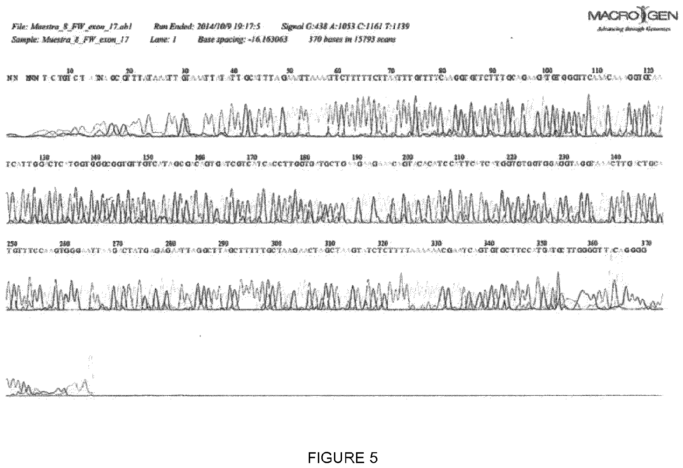

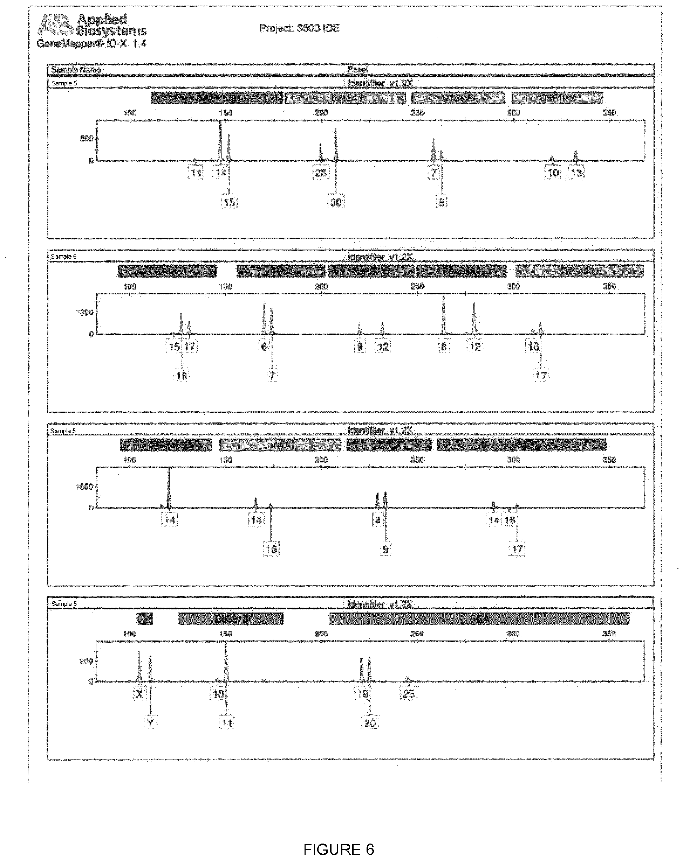

Figure 5 and 6 in annexes I and II, are images of test in the laboratory.

### DETAILED DESCRIPTION OF THE INVENTION

Figure 4 shows the sticker according to the present invention. The Sticker 10 includes in the following order:
1. A 50 microns crystal type BOOP
2. silicone
3. adhesive
4. a 60 microns white BOOP,
5. a layer of transparent material (to split the flap)
6. silicone
7. a 50 microns crystal type BOOP.

A longitudinal cut It is incorporated to the white BOOP layer in the middle and attached to the transparent layer so that when using it in the label it is removed to uncover it with a flap that facilitates without difficulty the top cover for a palm or digit hand posture of the hand or foot of any individual for the taking of epidermis and its later generic study.

The adhesive layer is water-based and includes a polymer that is used to stabilize fluid emulsions and stable suspensions of water base, whose dispersions are very transparent and have low sensitivity to ionic agents, with high tolerance to electrolytes.

On occasion, when limited amounts of DNA are available, as is the case in the samples of the finger epidermis, it is possible to resort to the technique of chain amplification of the DNA polymerase (PCR) to generate, from a sample, identical copies of a specific sequence and thus proceed to its sequencing, coding DNA can be obtained from the procedure to determine a pathology and genetic studies of specific pathologies and non-coding DNA which are low amounts but are sufficient to identity analysis for filiatory use. The extraction of the DNA of the adhesive of the reference is carried out in the form of swab and with a reagent provided by a commercial use kit (QIAamp) DNA investigator kit (QIAGEN).

In an embodiment, the present invention includes a kit for commercial having:
A) Adhesive sheets or sticker according to the present invention;
B) Sterile swabs (Tecnon);
C) 0.2 and 1 ml microtubes (Labnet and Axygen);
D) Tips (Delatalab);
E) Micropipettes P2, P20, P200, P1000 (Gilson);
F) )(QIAamp DNA investigator kit (QIAGEN);
G) Laminar flow table (MiLab);
H) Mini Spin centrifuge (Ependor);
I) Bio TDB-100;
J) Dry Block thermostat (BioSan);
K) NanoDrop 2000 (Thermo Scientific); and
L) Veriti 96well thermail cicler (Applied Biosystems)

Among the chemical properties of DNA is the verticality of the sequencer being amplified by the pcr system with chain reaction by polymerase, the hydration behavior is fundamentally being activated by the reagent of commercial use (QIAamp) DNA investigator kit (QIAGEN) causing electrophoretic mobility and sequential of nucleotide not being necessary in the disclosure of the characteristics of the bidder epidermal to avoid its identification.

The sequence of the amplified DNA by polymerase chain reaction comprises tandem repeats (str) locus. Acrylates: synthetic water-based resin with mixed ion exchange, proteases and fiber in which one side of the support is covered with an insoluble film.

The swab is with a buffer of the commercial type called tris with sodium chloride solution and then follows the molecular sieving. The result shows that traditional methods of the DNA extracted from the skin by the adhesive sheet can be used to obtain coding and non-coding DNA, being the polygenic used several amplifications per pcr in tandem for the subsequent risk reading of said pathology, this being a method that it allows reading.

### Technique

First, it was determined the efficiency and reproducibility of the collection method described by the latent DNA from the adhesive analyzing the quantity and quality of the same for gene analysis and hen the utility of that DNA for genetic diagnosis was determined in this specific case of Alzheimer's disease.

The sterile swabs used (tecnon) microtubes 0.2 and 1 ml (labnet and axygen), tips (deltalab).

Phase of extraction and characterization of DNA content of fingerprints, using the adhesive sheets (sticker) described above with a commercial kit of the type (QIAamp) DNA investigator kit (QIAGEN) were used with latent traces of 10 subjects (5 women and 5 men). Before taking the bidder, the subjects proceeded to wash their hands with soap and water, dried with paper and immediately captured the biological material under the described standards of latent prints. Each individual superimposed on the adhesive their fingerprints of each hand on a sticker and also the single take on a single thumb sticker of each hand, and the samples were coded to preserve identity.

For extraction of DNA, the swab was soaked with 50 ul of extraction solution of the kit (QIAamp) DNA investigator kit, following the manufacturer's instructions for use. The final elution of the DNA in 100 ul of elution buffer provided by the kit, previous quantification by molecular biology.

To measure the concentration of DNA in the samples, the standard system of quantification of nano drop TM DNA was used. The data obtained are included in the following table.

**TABLE 1**

| ADN amount by NanoDrop TM | | |
|---|---|---|
| Sample | Code | Concentration (ng/µl) |
| 1 | JDP-557 | -4,5 |
| 2 | AGL-805 | 0,62 |
| 3 | EAB-392 | -1,6 |
| 4 | RMP-968 | -3,3 |
| 5 | EZ-800 | -5,1 |
| 6 | PRH-840 | -4,6 |
| 7 | ALV-878 | -5,5 |
| 8 | MVRS-713 | -0,87 |
| 9 | CL-889 | -5,5 |
| 10 | SDO-828 | -6,3 |
| 11 buccal swap | PRH-840 | 4,5 |
| 12 Sticker without sample | | -4,2 |

The samples were also analyzed on agarose gels; they indicate that the DNA levels are below the detection sensitivity of the device.

Figure 1 shows the profile of the run of each sample, resulting in low levels of DNA but of very good quality since each sample is resolved in a continuous mark- up to approximately 5 k bases, suggesting a non-degraded DNA.

In order to corroborate the concentration of DNA in a precise way, the samples RMP-968 (4) and EZ-800 (5) were analyzed by RT-PCR for this, the samples were remitted to the lab. The report provided accounts for a quantification by using RTPCR using an Applied Biosystem 7500 cycler and the Quantifier Human DNA kit. The determinations indicate a DNA concentration of 0.1066 and 0.0815 ng / ul for sample 4 and 5 respectively. These data are very consistent.

Analysis of the quality of the DNA extracted from the sticker; aptitude for use for reaction in PCR. The technique of "polymerase chain reaction" or PCR (polymerase chain reaction) allows the generation of an exponential number of copies of specific DNA fragments using as a copy template DNA of any origin. An indispensable requirement for said amplification is that the DNA of the mold is not damaged or degraded; therefore, the technique can be used to determine the amount of an unknown DNA. For this purpose, the PCR technique amplifying the exon 17 of the app using the following primers:
5'_ATAACCTCATCCAAATGTCCCC-3 'and 5'-GTAACCCAAGCATCATGGAAG-3' (ChartierHarlin, Col.1991).

The PCR program used: denaturation, 3 minutes 94 degrees Celsius; 40 cycles of 30 seconds 92 degrees Celsius, 30 seconds 58 degrees centigrade, 30 seconds, 72 degrees Celsius.

The results of said amplification for finger samples are shown in the figure 2 and for samples of digit thumbs only in figure 3. In both cases, the amplified fragment corresponds to the expected size (399 bp). In the single thumbprint samples, it was amplified in 9 samples of 10, combining both hands. While for samples of multiple fingerprints, the amplification was 100% of the cases.

Figure 3 is an image showing agarose gels showing reactions of PCR from single thumbprints, where the top panel corresponds to the right thumb and lower panel corresponds to the left thumb.

Overall, the data from this analysis indicates that the method of thumbprint extraction allows to obtain a DNA of very good quality to perform manipulations later with diagnostic utility.

In order to verify the identity of the fragments generated by PCR and see their aptitude for the realization of a genetic diagnosis of Alzheimer's disease, we proceeded to sequencing of mimes. To do this, the materials amplified by PCR of exon 17 of the app (ChartierHarlin, Col.1991) were referred to a specialized laboratory. The reading of the sequences of each sample corresponds to the wild type of the gene in all cases. The reported sequence (see Annex I) is attached. Reading profiles and intensity of each base are observed (electroencephalograms) of excellent resolution in all cases.

To confirm if the samples do indeed come from a single DNA, the genetic profile of samples JDP-557 (1; F7898 / 1), EAB-392 (3; F7898 / 3). RPM-968 (4;

F7898 / 4) and EZ-800 (5; F7898 / 5) were analyzed by PCR for study of DNA polymorphism by analysis of STR loci, in the laboratory. The electroencephalograms provided attached (see Annex II) are compatible with the majority presence of a single DNA in each sample. A partial profile of sample 3 is also reported.

The data of this study indicates that the adhesive sheets (sticker) and the method used is suitable for collecting DNA in a sufficient quantity to be extracted and purified and allows PCR amplification and sequenced, by means of a commercial kit that can be used to genetic diagnosis and the study of the genetic profile by DNA polymorphism through analysis of STR loci.

It is therefore an object of the present invention to provide a sticker or adhesive sheet and associated method for obtaining human DNA for genetic analysis, characterized by the fact that the sticker comprises 6 sheets arranged one above the other, the first sheet being upper a BOPP of 50 microns, transparent of the crystal type, the second sheet with silicone treatment, one layer of acrylate adhesive, a third sheet another BOPP of 60 microns, white; a fourth transparent sheet, a fifth sheet with treatment of silicone and a sixth sheet BOPP of 50 microns, transparent of the crystal type, the associated method comprises to extract the samples a swab which was soaked with 50 ul of extraction solution of a commercially available DNA kit; because the final elution of the DNA in 100 ul of elution buffer provided by the kit, previous quantification by molecular biology; the PCR program used: denaturation, 3 minutes 94 degrees Celsius; 40 cycles of 30 seconds 92 degrees Celsius, 30 seconds 58 degrees Celsius, 30 seconds, 72 degrees Celsius and the PCR technique was used amplifying the exon 17 of the app using the following primers: 5'_ATAACCTCATCCAAPTGTCCCC-3 'and 5'- GTAACCCAAGCATCATGGAAG-3 '.

The epidermal cells captured in the adhesive sheets are dehydrated so that their DNA remains unchanged for storage.

## Claims

1. A sticker with adhesive sheet and method for obtaining human DNA for genetic analysis or diagnosis, **characterized by** the fact that the sticker comprises 6 sheets arranged one above the other,comprising:
Being a first top layer of 50 microns crystal type (BOOP);
a second layer with silicone treatment;
a sheet of acrylate adhesive;
a third layer of 60 microns white (BOOP)
a fourth layer of transparent material;
a fifth layer with silicone treatment, and;
a sixth layer of 50 microns crystal type (BOOP).

2. A sticker with adhesive sheet and method for obtaining human DNA , for genetic diagnosis according to claim 1 for the method comprising the step of:
for extracting a the swab was soaked with 50 ul of extraction solution from a commercially available DNA kit;
following the manufacturer's instructions for use.

3. A sticker with adhesive sheet and method for obtaining human DNA for genetic analysis being the method **characterized in that**, the final elution of the DNA in 100 ul of elution buffer provided by the kit, previous quantification by molecular biology.

4. A sticker with adhesive sheet and method for obtaining human DNA for genetic analysis being the method **characterized in that**, to measure the concentration of DNA in the samples, the standard system of quantification of nano drop TM DNA was used.

5. A sticker with adhesive sheet and method for obtaining human DNA for genetic analysis being the method **characterized in that**, the PCR program used: denaturation, 3 minutes 94 degrees Celsius; 40 cycles of 30 seconds 92 degrees Celsius, 30 seconds 58 degrees centigrade, 30 seconds, 72 degrees Celsius.

6. the method according to any one of the preceding claims, the PCR technique was used amplifying the exon 17 of the app using the following primers: 5'_ATAACCTCATCCAAPTGTCCCC-3 'and 5'-GTAACCCAAGCATCATGGAAG-3'.

7. A sticker with adhesive sheet and method for obtaining human DNA for genetic analysis or diagnosis according with claim 1; is incorporated in the comprising a longitudinal cut white BOOP layer in the middle and attached transparent layer so that when used on the label it is removed to uncover it with a flap that facilitates without difficulty the top cover for a palm or digit hand posture.
